# EUROPEAN PATENT SPECIFICATION

(11) **EP 2 010 166 B1**
(45) Date of publication and mention of the grant of the patent: **06.11.2013**
(21) Application number: 07755487.1
(22) Date of filing: 16.04.2007
(51) Int. Cl.: A61K 31/095, A61K 31/105, A61K 31/506, A61K 31/52, A61K 31/66, A61K 31/7072, A61P 31/18

(54) **ORGANIC DISULFIDE ANTIRETROVIRAL AGENTS**
ANTIRETROVIRALE MITTEL AUS ORGANISCHEM DISULFIDEN
AGENTS ANTIRETROVIRAUX AVEC DES DISULFIDES ORGANIQUES

(30) Priority: 17.04.2006 US 792431 P
(43) Date of publication of application: 07.01.2009
(73) Proprietor: The Government of the United States of America as represented by the Secretary of the Department of Health and Human Services, Rockville, MD 20852-3804 (US); Lovelace Respiratory Research Institute, Albuquerque, NM 87108 (US)
(72) Inventor: WALKER, Dale, M., Albuquerque, NM 87111 (US); WALKER, Vernon, E., Albuquerque, NM 87111 (US); POIRIER, Miriam, C., Bethesda, MD 20817 (US); SHEARER, Gene, M., Bethesda, MD 20817 (US)
(74) Representative: Valea AB
(86) International application number: PCT/US2007/009234
(87) International publication number: WO 2007/123868

(56) References cited:
- WO-A-94/22307
- US-A- 5 804 571
- US-A1- 2002 132 795
- US-B1- 6 489 312
- SANTINI V ET AL: "THE POTENTIAL OF AMIFOSTINE: FROM CYTOPROTECTANT TO THERAPEUTIC AGENT" HAEMATOLOGICA, FONDAZIONE FERRATA STORTI, ROME, IT, vol. 84, 1 January 1999 (1999-01-01), pages 1035-1042, XP000938345 ISSN: 0390-6078
- GÜL ÖZCAN ARICAN: "Cytoprotective effects of amifostine and cysteamine on cultured normal and tumor cells treated with paclitaxel in terms of mitotic index and 3H-thymidine labeling index" CANCER CHEMOTHERAPY AND PHARMACOLOGY, SPRINGER-VERLAG, BE, vol. 56, no. 2, 1 August 2005 (2005-08-01), pages 221-229, XP019334184 ISSN: 1432-0843
- CAPIZZI ET AL: "The preclinical basis for broad-spectrum selective cytoprotection of normal tissues from cytotoxic therapies by amifostine (Ethyol(R))" EUROPEAN JOURNAL OF CANCER, PERGAMON PRESS, OXFORD, GB, vol. 32, 1 January 1996 (1996-01-01), pages S5-S16, XP005055733 ISSN: 0959-8049

## Description

### FIELD OF THE INVENTION

A compound of formula II for use in the prevention or treatment of human immunodeficiency virus infection by administering an effective amount said compound to an individual in need is provided.

### BACKGROUND OF THE INVENTION

Human immunodeficiency virus (HIV) has been identified as the etiological agent responsible for acquired immune deficiency syndrome (AIDS), a fatal disease characterized by destruction of the immune system and the inability to fight off life threatening opportunistic infections. According to estimates from the UNAIDS/WHO AIDS Epidemic Update (December 2005), 38.0 million adults and 2.3 million children were living with HIV at the end of 2005. This is more than 50% higher than the figures projected by WHO in 1991 on the basis of the data then available. During 2005, some 4.9 million people became infected with the human immunodeficiency virus. The year also saw 3.1 million deaths from AIDS - a high global total, despite antiretroviral (ARV) therapy, which reduced AIDS-related deaths among those who received it. Deaths among those already infected will continue to increase for some years even if prevention programs manage to cut the number of new infections to zero. However, with the HIV-positive population still expanding, the annual number of AIDS deaths can be expected to increase for many years.

There are currently a number of antiretroviral drugs available to combat the infection. These drugs can be divided into three classes based on their mode of action. In particular, the protease inhibitors (PIs), including but not limited to saquinavir, indinavir, ritonavir, nelfinavir, and amprenavir, are competitive inhibitors of the aspartyl protease expressed by HIV. The nucleoside reverse transcriptase inhibitors (NRTIs), including but not limited to zidovudine, zalcitabine, didanosine, stavudine, lamivudine, and abacavir, are nucleoside analogs that behave as substrate mimics to halt viral cDNA synthesis. The non-nucleoside reverse transcriptase inhibitors (NNRTI), including but not limited to nevirapine, delavirdine, and efavirenz, inhibit the synthesis of viral cDNA via a non-competitive or uncompetitive mechanism. Used alone, many of these drugs are effective in reducing retroviral replication, but no single drug permanently inhibits retrovirus growth and most allow the retrovirus to develop resistance. Current standard-of-care therapies, termed Highly Active Antiretroviral Therapy (HAART), which have proven very effective at both reducing retroviral replication and suppressing the emergence of resistance in patients, comprise drug "cocktails" that typically include two NRTIs, with a PI or an NNRTI. The total number of people living with HIV continues to rise in high-income countries, such as the United States, largely due to widespread access to antiretroviral treatment, which prolongs the life of individuals living with HIV and AIDS.

Unfortunately, not all patients are responsive to the above-referenced antiretroviral therapies. Treatment failure in most cases is caused by the emergence of retroviral resistance, which is typically caused by a high retroviral mutation rate. Under these circumstances, incomplete retroviral suppression may be the result of insufficient drug potency, poor compliance to complicated drug regiments, and intrinsic pharmacological barriers to exposure. For example, while nucleoside reverse transcriptase inhibitors are essential components of the highly-successful HAART combination therapies, they are, however, also genotoxins that become incorporated into host DNA by 5'-phosporylation. Lacking a ribose 3'OH group, the incorporated NRTIs terminate DNA replication inducing chromosomal damage and mutagenesis in many experimental systems, and tumorigenesis in rodents (Poirier et al., Tox Appl Pharmacol 199:151, 2004).

### SUMMARY OF THE INVENTION

There is a clear need for new antiretroviral agents and combination therapies to suppress HIV replication even further than currently available therapies. Even more desirable are new antiretroviral agents or ombination therapies capable of reducing HIV levels below detectable levels. This is accomplished by the invention as defined hereinafter.

Amifostine (referred to as "WR-2721 "), the FDA-licensed drug Ethyol, is used clinically to protect against toxicity induced by chemotherapy and radiation-therapy (Grdina et al., Drug Met Drug Interact 16:237, 2000). Amifostine is a phosphorylated pro-drug that is metabolized by alkaline phosphatase to the reduced free thiol active metabolite (referred to as "WR-1065'); oxidation of WR-1065 leads to formation of a disulfide (referred to as "WR-33278"). These reactions can be depicted schematically as follows:

| |
|---|
| Amifostine (WR-2721) |
| H₂N(CH₂)₃NH(CH₂)₂SPO₃H₂ |
| + |
| Alkaline Phosphatase |
| ↓ |
| WR-1065 |
| H₂N(CH₂)₃NH(CH₂)₂SH |
| Reduction ↑ Oxidation ↓ |
| WR-33278 |
| H₂N(CH₂)₃NH(CH₂)₂SS(CH₂)₂NH(CH₂)₃NH₂ |

Mechanisms by which amifostine provides a cytoprotective effect include thiol-mediated free radical scavenging by direct contact or through binding to transcription factors (NFκB, Asp-1, p53) with induction of MnSOD and other relevant proteins, as well as electrostatic binding to, and (polyamine-like) stabilization of the DNA helix by inhibition of Topo II, allowing enhanced DNA repair, resulting in reduced mutagenesis.

Structural similarities can be observed between spermine (chemical formula H₂N(CH₂)₃NH(CH₂)₂(CH₂)₂NH(CH₂)₃NH₂), WR-1065, and WR-33278. Spermine is associated with nucleic acids and is thought to stabilize helical structure, particularly in viruses. WR1065 appears to function as an analog of the polyamine spermine, and to compete with spermine for sites on DNA, and probably also on other nucleic acids and proteins. Thus, WR-1065 and other compounds discussed below, may function as spermine or other polyamine analogs, and may mimic the anti-HIV and polyamine-like activities of WR-1065.

The thiol (WR-1065) and the disulfide (WR-33278) metabolites of amifostine are believed to be responsible for most of the cytoprotective and radioprotective properties of amifostine. Amifostine is taken up by cells through a combination of passive and active transport processes where it is metabolized to its active forms; these metabolites participate in a number of functions. These functions include, but are not limited to, (i) protection against radiation induced cytotoxicity and cell killing, (ii) protection against radiation-induced mutagenesis/carcinogenesis, (iii) modulation of topoisomerase I and topoisomerase II alpha activities, (iv) modulation of conformational changes in chromatid structure, (v) inhibition of cell cycle progression, (vi) inhibition of endonuclease activity, (vii) competition with spermine in polyamine transport systems, (viii) induction and repression of gene expression (effect dependent upon the particular gene). Other reported functions include detoxifying cisplatin and other alkylating agents, scavenging free radicals, modulating apoptosis, and modifying the activity of specific enzymes/proteins. The cytoprotective effects of amifostine appear to be dependent upon a number of factors including, but not limited to, oxygen tension, pH, gene status (including the presence of a functional p53 gene), and enzyme status (including the expression of alkaline phosphatase in the cell membrane, and the functionality of superoxide dismutase). Differences in these factors appear to be responsible for the differential cytoprotective effect mediated by amifostine between tumor cells and normal, nontumorigenic tissue. It has been discovered that amifostine is particularly effective in inhibiting replication of HIV.

A compound for use in treating or preventing a human immunodeficiency virus infection in a patient in need thereof is provided, comprising the step of: administering to the patient an effective antiretroviral amount of a compound or a pharmaceutically acceptable salt or solvate thereof in a unit dosage form, wherein the compound is of Formula (I) or Formula (II): 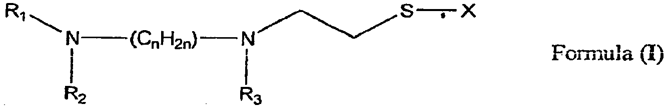 and 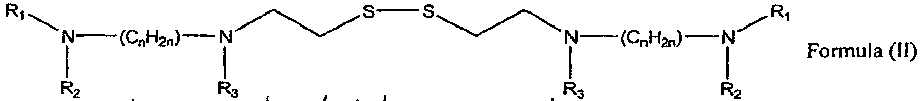 The invention is directed to compounds of Formula II for use in preventing or treating a human immunodeficiency virus infection. wherein X is selected from the group consisting of hydrogen and a leaving group, wherein each of R₁, R₂, and R₃ is independently selected from hydrogen and C₁₋₆ alkyl, and wherein n is an integer of from 1 to 10.

In an embodiment of the invention, the medicament comprises from about 500 mg to about 700 mg of the compound.

In an embodiment of the invention, the medicament further comprises an effective antiretroviral amount of at least one nucleoside reverse transcriptase inhibitor.

In an embodiment of the invention, the medicament comprises from 100 mg to 300 mg of the compound.

In an embodiment of the invention, the medicament when administered once yields a peak intracellular concentration of less than 30 nanomolar.

A compound of formula (I) wherein X is H may be provided.

In an embodiment of the invention, the compound is of formula (II).

In an embodiment of invention, the compound is

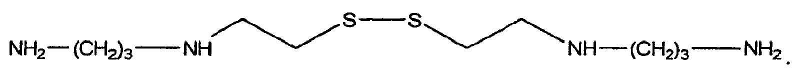

In an embodiment of the invention, the use further comprises a Cu-dependent amine-oxidase blocker.

The Cu-dependent amine-oxidase blocker may be aminoguanidine.

In an embodiment of the invention, the use further comprises a reducing agent selected from the group consisting of vitamin C, vitamin E, glucose, mannose, galactose, xylose, ribose, arabinose, and combinations thereof.

In an embodiment of the invention, the nucleoside reverse transcriptase inhibitors are selected from the group consisting of zidovudine, didanosine, stavudine, zalcitabine, lamivudine, abacavir, and combinations thereof. At least one of the nucleoside reverse transcriptase inhibitors can be zidovudine, e.g., administered to the patient at a daily dosage of from about 300 mg/day to about 400 mg/day.

### DETAILED DESCRIPTION OF THE PREFERRED EMBODIMENT

The following description and examples illustrate some exemplary embodiments of the disclosed invention in detail. Those of skill in the art will recognize that there are numerous variations and modifications of this invention that are encompassed by its scope. Accordingly, the description of a certain exemplary embodiment should not be deemed to limit the scope of the present invention.

### Amifostine

Amifostine is an organic thiophosphate which selectively protects normal tissues but not tumors against cytotoxicity of ionizing radiations, DNA-binding chemotherapeutic agents (*e.g*., classical alkylating agents such as cyclophosphamide and non-classical alkylating agents such as mitomycin-C and platinum analogs). Amifostine is a prodrug that is dephosphorylated to the active metabolite, the free thiol form, by alkaline phosphatase and exits the bloodstream rapidly.

The compounds of Formula I or II, including amifostine and its derivatives and analogs, are particularly effective antiretroviral compounds for use in inhibiting replication of HIV.

Amifostine is marketed under the name Ethyol® by Schering-Plough Pty Ltd. It has the chemical name S-2-(3-aminopropyl)aminoethyl phosphorothioic acid and the structure:

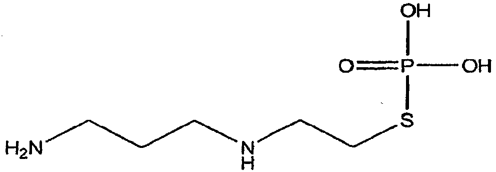

An antiretroviral compound for use in inhibiting replication of HIV includes the free thiol form of amifostine. The reduced free thiol form has the chemical name 2-(3-aminopropylamino)ethanethiol and the following structure:

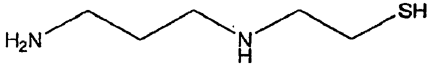

Phosphonol (referred to as "WR-3689") is structurally similar to amifostine, the only difference being the presence of a terminal methyl group. Phosphonol has the chemical name S-2-(3-(methylamino)propylamino)ethyl phosphorothioic acid and the following structure:

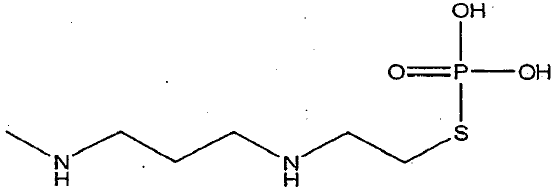

The free thiol form of phosphonol has the chemical name 2-(3-(methylamino)propylamino) ethanethiol and the following structure:

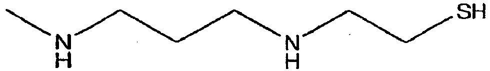

The compounds disclosed include prodrug forms of the above-described free thiol forms of amifostine, phosphonol, and analogs thereof. Such prodrugs include compounds of the structure: 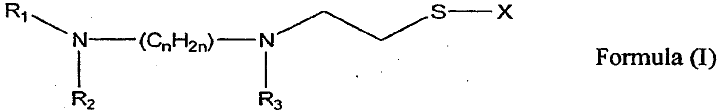 wherein each of R₁, R₂, and R₃ is independently selected from hydrogen and lower alkyl, wherein -(CₙH₂ₙ)- is lower alkyl and n is 1, 2, 3, 4, 5, 6, 7, 8, 9, or 10 and wherein X is hydrogen or a suitable leaving group. Suitable leaving groups include phosphonate groups (*e.g*., -PO₃H₂), or acetyl, isobutyryl, pivaloyl, or benzoyl groups; however, any other suitable leaving group can be employed that yields the active free thiol form of the compound when metabolized *in vivo.* Other classes of leaving groups include alkyl groups, *e.g*., -(C₁₋₆ alkyl), and keto groups, *e.g*., -C(=O)-(C₁₋₆ alkyl) or -C(=O)-(C₆₋₁₈ aryl).

The term "lower alkyl" as used herein is a broad term, and is to be given its ordinary and customary meaning to a person of ordinary skill in the art (and is not to be limited to a special or customized meaning), and refers without limitation to a straight chain or branched chain, acyclic or cyclic, saturated aliphatic hydrocarbon containing 1, 2, 3, 4, 5, 6, 7, 8, 9, or 10 carbon atoms. Saturated straight chain lower alkyls include methyl, ethyl, n-propyl, n-butyl, n-pentyl, and n-hexyl; while representative saturated branched chain alkyls include isopropyl, *sec*-butyl, isobutyl, *tert*-butyl, and isopentyl. The term "aryl" as used herein is a broad term, and is to be given its ordinary and customary meaning to a person of ordinary skill in the art (and is not to be limited to a special or customized meaning), and refers without limitation to an aromatic carbocyclic moiety such as phenyl or naphthyl, including mono-, di-, and poly-homocyclic aromatic ring systems (*e.g*., C₆₋₁₈ aryl).

The compounds of Formula I or II can be provided in prodrug form. As used herein, a "prodrug" refers to a compound that may not necessarily be pharmaceutically active, or may only exhibit minimal activity, but that is converted into an active (or more active) drug upon *in vivo* administration. The prodrug can be designed to alter the metabolic stability or the transport characteristics of a drug, to mask side effects or toxicity, to improve the flavor of a drug or to alter other characteristics or properties of a drug. Prodrugs are often useful because they may be easier to administer than the parent drug. They may, for example, be bioavailable by oral administration whereas the parent drug is not. The prodrug may also have better solubility than the active parent drug in pharmaceutical compositions. An example, without limitation, of a prodrug would be, a compound of formula (I) wherein X is a leaving group that is cleaved *in vivo* to yield the free thiol. By virtue of knowledge of pharmacodynamic processes and drug metabolism *in vivo*, those skilled in the art, once a pharmaceutically active compound is known, can design prodrugs of the compound (see, *e.g.* Nogrady (1985) Medicinal Chemistry A Biochemical Approach, Oxford University Press, New York, pages 388-392). The prodrug forms described above are metabolized into active thiols of the formula: 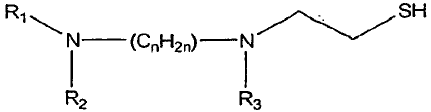 wherein each of R₁, R₂, R₃, -(CₙH₂ₙ)-, and n is as defined above. In an embodiment, -(CₙH₂ₙ)- is a straight alkyl chain having three carbon atoms. It is also particularly preferred that R₂ and R₃ are both hydrogen, and that R₁ is hydrogen or methyl.

In addition to the thiols, certain disulfide forms also exhibit antiretroviral activity, for example, disulfides of the following structures: 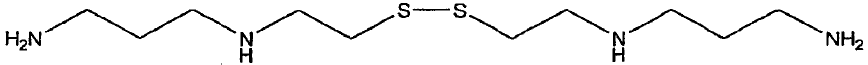 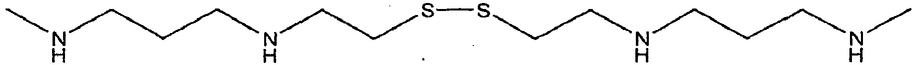 can also be employed as antiretroviral agents.

The compounds of Formula I or II are particularly effective antiretroviral agents for monotherapeutic or combined-therapeutic use in treating HIV infection. The compounds of Formula I or II are generally administered at dosages equal to or less than the oral radioprotective dosage of amifostine (*e.g*., 1456 mg total dose, or 910 mg/m² for a 60 kg body weight (BW) adult human) or phosphonol (*e.g*., 725 mg/m² for a 60 kg BW adult human). In adults undergoing chemotherapy, the recommended starting dose of amifostine is 910 mg/m² for a 60 kg BW adult human administered once daily as a 15-minute i.v. infusion, starting 30 minutes prior to chemotherapy. Similar dosing regimens can be employed for use of the compounds of Formula I or II when used as antiretroviral agents. Dosages can be converted from mg/m² to total mg or mg/kg BW (see, *e.g.,* Freireich et al. (1966), Cancer Chemother. Reports, 50 (4) 219-244) as in Table 1.

**Table 1.**

| **Species** | **Body Wt. (Kg)** | **Body Surface area (m²)** | **Km factor** |
|---|---|---|---|
| Mouse | 0.2 | 0.0066 | 3.0 |
| Rat | 0.15 | 0.025 | 5.9 |
| Monkey | 3.0 | 0.24 | 12 |
| Dog | 8.0 | 0.40 | 20 |
| Human child | 20.0 | 0.80 | 25 |
| Human adult | 60.0 | 1.60 | 37 |

It is generally preferred to administer amifostine (or other compounds of Formula I or II) at dosages of 910 mg/m² or less for a 60 kg BW adult human. This dosage is equivalent to 24.3 mg/kg BW for a 60 kg BW adult human being (or a total dose of 1456 mg for a 60 kg BW adult, as described above); however, in certain embodiments it can be desirable to administer the compounds of Formula I or II at higher dosage levels. For example, children have been given up to a 2700 mg/m² total dose of amifostine prior to administration of a chemotherapeutic agent. In some individuals such high doses are associated with side effects. A dose of 740 mg/m² amifostine (1148 mg total, for a 60 kg BW human adult) is associated with fewer side effects (List et al. (1997), Blood 90(9): 3364-9), and is thus generally preferred. For daily dosing, 200-340 mg/m² of amifostine (544 mg total dose for a 60 kg BW adult) is generally preferred (Schuchter (1996), Semin Oncol 23(4 Suppl 8): 40-3; Santini et al. (1999), Haematologica 84(11): 1035-42). Amifostine, given by injection at 500-910 mg/m², has a plasma T_{1/2} of ~10 minutes. The metabolite WR-1065 has a peak plasma level of ∼100 µM, with a T_{1/2} of ≥2 hr, widespread tissue distribution and high bioavailability.

Rodent studies suggest the use of higher dosages. For example, the maximally tolerated dose (MTD) for WR-2721 in mice was 432 mg/kg BW administered i.p. and 720 mg/kg BW administered p.o., and the 100% effective radioprotective dose was about one half of the MTD. For phosphonol, the MTD was 893 mg/kg BW administered i.p. and 1488 mg/kg BW administered p.o., and the 100% effective radioprotective dose was about one half of the MTD. All of the aminothiols have MTDs in rodents of greater than 400 mg/kg BW.

Table 2 provides plasma concentrations of amifostine metabolites (see Geary et al., Res. Comm. Chem. Path. Pharmacol., 65(2), 147-159 (1989)) and phosphonol metabolites (see Buckpitt *et al.,* Contract #DAMD 17-86-C-6177, reference obtained from Dr. D. Grdina, personal communication) after duodenal administration of 150 mg/kg BW of each drug in rhesus macaques. These data may be useful in estimating plasma concentrations in humans.

**Table 2.**

| **Drug** | **Time after Administration (h)** | **Concentration (µg/ml)** |
|---|---|---|
| WR-1065¹ | 1 | 6.21 |
| WR-1065¹ | 2 | 4.14 |
| WR-1065¹ | 6 | 2.07 |
| Phosphonol² | 1 | 11.75 |
| Phosphonol² | 2 | 17.08 |
| Phosphonol² | 6 | 15.50 |
| Note: 40 µM WR-1065 = 8.28 µg/ml | | |

While it is generally preferred to formulate amifostine or the compounds of Formula I or II for oral administration, the compounds of Formula I or II can be formulated so as to allow them to be administered by other routes, as discussed below. It can be desirable in certain embodiments to formulate amifostine for intravenous administration in order to maximize efficacy. Because of the structural similarities between amifostine and phosphonol, especially the similarities in the sulfhydryl ends of the molecules, phosphonol is expected to behave in a manner similar to amifostine rather than WR-151327. Phosphonol may not be quite as efficacious as amifostine, based upon the work of Gutschow *et al.,* who found lower activity when a methyl group was substituted for a hydrogen atom in a position similar to that of the methyl group of phosphonol that distinguishes its structure from that of amifostine (Gutschow et al. (1995), Pharmazie 50(10): 672-5). The overall structures of the compounds tested by Gutschow *et al*. are significantly different from the structures of the Formula I or II, and it is expected that phosphonol will be significantly more efficacious than WR-151327.

### Combination Therapies with Antiretroviral Drugs

Amifostine, its analogs, and its derivatives can be administered in combination with other antiretroviral agents employed to treat HIV infection. One of the benefits of such combination therapies is that lower doses of the other antiretroviral agents can potentially be administered while achieving a satisfactory level of antiretroviral efficacy. Such lower dosages can be particularly advantageous for antiretroviral drugs known to have genotoxicity and mitochondrial toxicity, and may attenuate resistance to the antiretroviral drug.

The most common antiretroviral NRTIs that can be used in conjunction with the compounds of Formula I or II include, but are not limited to, zidovudine, and lamivudine - nucleoside analogs active against HIV that are typically used together. Zidovudine (also referred to as azidothymidine (AZT)) is marketed under the trade name Retrovir®,and lamivudine (also referred to as thiacytidine (3TC)) is marketed under the trade name Epivir®. Both drugs are sold by GlaxoSmithKline separately and together in the clinical formulation Combivir®. Intracellularly, both drugs are phosphorylated to the active 5'-triphosphate metabolites, which are used in place of normal nucleotides thymidine and cytosine for DNA replication. Once incorporated into DNA, the analogs do not support further DNA replication, and are therefore obligate DNA chain terminators. The principal mode of antiretroviral action is inhibition of reverse transcription, however they can also be weak inhibitors of the nuclear DNA polymerase a and strong inhibitors of the mitochondrial DNA polymerase y.

Use of the NRTI zidovudine has been associated with hematologic toxicity including neutropenia and anemia, particularly in patients with advanced HIV. Prolonged use of NRTIs has been associated with mitochondrial toxicity evidenced by heart and skeletal myopathies, which can occasionally progress to life-threatening pancreatitis or lactic acidosis with severe hepatomegaly and steatosis. Administering compounds of the Formula I or II in combination with NRTIs may desirably enable the dosage of NRTIs to be minimized. The compounds of the Formula I or II, when administered in combination with NRTIs, can also mitigate the genotoxic effects of NRTI therapy.

The compounds of the Formula I or II when employed in combination with NRTIs can be administered at any appropriate time, and in any appropriate fashion, e.g., before, during, or after administration of the NRTI, in the same or different unit dosage form as the NRTI, in combination with other therapeutic agents, and in any suitable unit dosage form (e.g., oral, intravenous, etc.).

In conventional HIV infection treatments, NRTIs are administered in an oral dosage form (*e.g*., capsules, tablets, oral solution). For example, zidovudine is generally administered at 600 mg/day in divided doses, alone or in combination with PIs and/or NNRTIs. Dosages of zidovudine when administered by injection in conventional HIV therapies are typically from about 1 to 2 mg/kg of BW, injected every four hours five to six times a day. Dosages for FDA approved NRTIs are as follows.

**Table 3.**

| **Nucleoside Analog** | **Normal Adult Dosage** |
|---|---|
| Abacavir (Ziagen™ | 300 mg PO BID |
| Didanosine (Videx® Videx® EC) | 200 mg PO BID or 400 mg PO QD (Tablets) |
| Lamivudine (Epivir®) | 150 mg PO BID |
| Stavudine (Zerit®) | 30-40 mg PO BID |
| Zalcitabine (Hivid®) | 0.25-0.75 mg PO BID |
| Zidovudine (Retrovir®) | 300 mg BID PO |

**Table 4.**

| **Nucleoside Analog** | **Pediatric Dosage** |
|---|---|
| Didanosine | 120 mg.m2 PO q12h |
| Lamivudine | 4 mg/kg (Maximum 150 mg) PO BID |
| Zidovudine | 160 mg/m2 PO q8h |

In addition to the NRTIs discussed above, other NRTIs can be used in combination with the compounds of Formula I or II, including NRTIs currently under development.

While WR-1065 was initially developed as a cytoprotective and anti-mutagenic agent to protect cells and organs from the deleterious effects of nucleoside analogs or NRTIs, it has broad clinical applicability in that it also exhibits anti-viral effects and can be used in conjunction with nucleoside analogs as a protective agent during the treatment of other diseases/conditions for which nucleoside analogs have efficacy.

Use of the compounds of Formula I or II with zidovudine has been tested in cell culture experiments, and it is anticipated that the compounds of Formula I or II can also be effectively employed with other nucleoside analogs, including, but not limited to, didanosine, lamivudine, stavudine, and abacavir.

In addition to the agents used to treat HIV infection as described above, the compounds of Formula I or II can also be administered in combination with other agents. These include NNRTIs and PIs that are components in HAART therapy, as well as other aminothiols or other antiretroviral drugs.

### Pharmaceutical Compositions Comprising Amifostine and Analogs Thereof

The compounds of Formula I or II (including derivatives, isomers, metabolites, prodrugs, or pharmaceutically acceptable esters, salts, and solvates thereof) can be incorporated into a pharmaceutically acceptable carrier, including incorporation into nanoparticles, for administration to an individual having an HIV infection or AIDS, or can be administered prophylactically to prevent HIV infection or AIDS. The compounds of Formula I or II can be employed as the sole agent in the prevention or treatment of HIV or AIDS, or two or more such compounds can be employed, optionally in combination with other therapeutic agents, *e.g*., conventional or newly developed drugs employed in the treatment of AIDS or HIV, or other retroviral infections.

### Nanoparticulate Forms

In some embodiments, the compounds of Formula I or II are provided in nanoparticulate form, e.g., drug nanoparticles in solid form (e.g., as a tablet or capsule) or nanoparticles in liquid suspension (e.g., for oral or intravenous administration). Powders comprising nanoparticulate drug can be made by spray-drying aqueous dispersions of a nanoparticulate drug and a surface modifier to form a dry powder which consists of aggregated drug nanoparticles. An aqueous dispersion of drug and surface modifier can contain a dissolved diluent such as lactose or mannitol which, when spray dried, forms embedded drug nanoparticles. In one embodiment, compositions are provided containing nanoparticles which have an effective average particle size of less than about 1000 nm, more preferably less than about 400 nm, less than about 300 nm, less than about 250 nm, less than about 100 nm, or less than about 50 nm, as measured by light-scattering methods. By "an effective average particle size of less than about 1000 nm" it is meant that at least 50% of the drug particles have a weight average particle size of less than about 1000 nm when measured by light scattering techniques. Preferably, at least 70% of the drug particles have an average particle size of less than about 1000 nm, more preferably at least 90% of the drug particles have an average particle size of less than about 1000 nm, and even more preferably at least about 95% of the particles have a weight average particle size of less than about 1000 nm.

The compounds of Formula I or II can be provided in the form of a spray-dried powder, either alone or combined with a freeze-dried nanoparticulate powder. Spray-dried or freeze-dried nanoparticulate powders can be mixed with liquid or solid excipients to provide unit dosage forms suitable for administration. Freeze dried powders of a desired particle size can be obtained by freeze drying aqueous dispersions of drug and surface modifier, which additionally contain a dissolved diluent such as lactose or mannitol.

Milling of aqueous drug to obtain nanoparticulate drug may be performed by dispersing drug particles in a liquid dispersion medium and applying mechanical means in the presence of grinding media to reduce the particle size of the drug to the desired effective average particle size. The particles can be reduced in size in the presence of one or more surface modifiers. Alternatively, the particles can be contacted with one or more surface modifiers after attrition. Other compounds, such as a diluent, can be added to the drug/surface modifier composition during the size reduction process. Dispersions can be manufactured continuously or in a batch mode.

Another method of forming a nanoparticle dispersion is by microprecipitation. This is a method of preparing stable dispersions of drugs in the presence of one or more surface modifiers and one or more colloid stability enhancing surface active agents free of any trace toxic solvents or solubilized heavy metal impurities. Such a method comprises, for example, (1) dissolving the drug in a suitable solvent with mixing; (2) adding the formulation from step (1) with mixing to a solution comprising at least one surface modifier to form a clear solution; and (3) precipitating the formulation from step (2) with mixing using an appropriate nonsolvent. The method can be followed by removal of any formed salt, if present, by dialysis or diafiltration and concentration of the dispersion by conventional means.

In a non-aqueous, non-pressurized milling system, a non-aqueous liquid having a vapor pressure of about 1 atm or less at room temperature and in which the drug substance is essentially insoluble may be used as a wet milling medium to make a nanoparticulate drug composition. In such a process, a slurry of drug and surface modifier may be milled in the non-aqueous medium to generate nanoparticulate drug particles. Examples of suitable non-aqueous media include ethanol, trichloromonofluoromethane, (CFC-11), and dichlorotetafluoroethane (CFC-114). An advantage of using CFC-11 is that it can be handled at only marginally cool room temperatures, whereas CFC-114 requires more controlled conditions to avoid evaporation. Upon completion of milling the liquid medium may be removed and recovered under vacuum or heating, resulting in a dry nanoparticulate composition.

In a non-aqueous, pressurized milling system, a non-aqueous liquid medium having a vapor pressure significantly greater than 1 atm at room temperature may be used in the milling process to make nanoparticulate drug compositions. The milling medium can be removed and recovered under vacuum or heating to yield a dry nanoparticulate composition.

Spray drying is a process used to obtain a powder containing nanoparticulate drug particles following particle size reduction of the drug in a liquid medium. In general, spray-drying may be used when the liquid medium has a vapor pressure of less than about 1 atm at room temperature. A spray-dryer is a device which allows for liquid evaporation and drug powder collection. A liquid sample, either a solution or suspension, is fed into a spray nozzle. The nozzle generates droplets of the sample within a range of about 20 to about 100 µm in diameter which are then transported by a carrier gas into a drying chamber. The carrier gas temperature is typically between about 80 and about 200° C. The droplets are subjected to rapid liquid evaporation, leaving behind dry particles which are collected in a special reservoir beneath a cyclone apparatus.

If the liquid sample consists of an aqueous dispersion of nanoparticles and surface modifier, the collected product will consist of spherical aggregates of the nanoparticulate drug particles. If the liquid sample consists of an aqueous dispersion of nanoparticles in which an inert diluent material was dissolved (such as lactose or mannitol), the collected product will consist of diluent (e.g., lactose or mannitol) particles which contain embedded nanoparticulate drug particles. The final size of the collected product can be controlled and depends on the concentration of nanoparticulate drug and/or diluent in the liquid sample, as well as the droplet size produced by the spray-dryer nozzle.

In some instances it may be desirable to add an inert carrier to the spray-dried material to improve the metering properties of the final product. This may especially be the case when the spray dried powder is very small (less than about 5 µm) or when the intended dose is extremely small, whereby dose metering becomes difficult. In general, such carrier particles (also known as bulking agents) are too large to be delivered to the lung and simply impact the mouth and throat and are swallowed. Such carriers typically consist of sugars such as lactose, mannitol, or trehalose. Other inert materials, including polysaccharides and cellulosics, may also be useful as carriers.

Sublimation can be employed to obtain a nanoparticulate drug composition. Sublimation avoids the high process temperatures associated with spray-drying. In addition, sublimation, also known as freeze-drying or lyophilization, can increase the shelf stability of drug compounds, particularly for biological products. Sublimation involves freezing the product and subjecting the sample to strong vacuum conditions. This allows for the formed ice to be transformed directly from a solid state to a vapor state. Such a process is highly efficient and, therefore, provides greater yields than spray-drying. The resultant freeze-dried product contains drug and modifier(s).

### Reducing Agents

Because certain of the compounds of Formula I or II can be sensitive to oxidation, it can be desirable to administer the compounds in combination with reducing agents including, but not limited to, vitamin C and vitamin E. Other reducing agents include organic aldehydes, hydroxyl-containing aldehydes, and reducing sugars such as glucose, mannose, galactose, xylose, ribose, and arabinose. Other reducing sugars containing hemiacetal or keto groupings can be employed, for example, maltose, sucrose, lactose, fructose, and sorbose. Other reducing agents include alcohols, preferably polyhydric alcohols, such as glycerol, sorbitol, glycols, especially ethylene glycol and propylene glycol, and polyglycols such as polyethylene and polypropylene glycols.

### Pharmaceutical Compositions and Unit Dosage Forms

The terms "pharmaceutically acceptable salts" and "a pharmaceutically acceptable salt thereof" as used herein are broad terms, and are to be given their ordinary and customary meaning to a person of ordinary skill in the art (and are not to be limited to a special or customized meaning), and refer without limitation to salts prepared from pharmaceutically acceptable, non-toxic acids or bases. Suitable pharmaceutically acceptable salts include metallic salts, *e.g.*, salts of aluminum, zinc, alkali metal salts such as lithium, sodium, and potassium salts, alkaline earth metal salts such as calcium and magnesium salts; organic salts, *e.g*., salts of lysine, N,N'-dibenzylethylenediamine, chloroprocaine, choline, diethanolamine, ethylenediamine, meglumine (N-methylglucamine), procaine, and tris; salts of free acids and bases; inorganic salts, *e.g*., sulfate, hydrochloride, and hydrobromide; and other salts which are currently in widespread pharmaceutical use and are listed in sources well known to those of skill in the art, such as, for example, The Merck Index. Any suitable constituent can be selected to make a salt of the therapeutic agents discussed herein, provided that it is non-toxic and does not substantially interfere with the desired activity. In addition to salts, pharmaceutically acceptable precursors and derivatives of the compounds can be employed. Pharmaceutically acceptable amides, lower alkyl esters, and protected derivatives of the compounds of Formula II can also be suitable for use in the prevention or treatment of a human immunodeficiency virus infection. While it may be possible to administer the compounds of the preferred embodiments in the form of pharmaceutically acceptable salts, it is generally preferred to administer the compounds in neutral form.

It is generally preferred to administer the compounds of Formula I or II in an intravenous or subcutaneous unit dosage form; however, other routes of administration are also contemplated. Contemplated routes of administration include but are not limited to oral, parenteral, intravenous, and subcutaneous. The compounds of Formula I or II can be formulated into liquid preparations for, *e.g.*, oral administration. Suitable forms include suspensions, syrups, elixirs, and the like. Particularly preferred unit dosage forms for oral administration include tablets and capsules. Unit dosage forms configured for administration once a day are particularly preferred; however, in certain embodiments it can be desirable to configure the unit dosage form for administration twice a day, or more.

The pharmaceutical compositions are preferably isotonic with the blood or other body fluid of the recipient. The isotonicity of the compositions can be attained using sodium tartrate, propylene glycol or other inorganic or organic solutes. Sodium chloride is particularly preferred. Buffering agents can be employed, such as acetic acid and salts, citric acid and salts, boric acid and salts, and phosphoric acid and salts. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. In certain embodiments it can be desirable to maintain the active compound in the reduced state. Accordingly, it can be desirable to include a reducing agent, such as vitamin C, vitamin E, or other reducing agents as are known in the pharmaceutical arts, in the formulation.

Viscosity of the pharmaceutical compositions can be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the thickening agent selected. An amount is preferably used that will achieve the selected viscosity. Viscous compositions are normally prepared from solutions by the addition of such thickening agents.

A pharmaceutically acceptable preservative can be employed to increase the shelf life of the pharmaceutical compositions. Benzyl alcohol can be suitable, although a variety of preservatives including, for example, parabens, thimerosal, chlorobutanol, or benzalkonium chloride can also be employed. A suitable concentration of the preservative is typically from about 0.02% to about 2% based on the total weight of the composition, although larger or smaller amounts can be desirable depending upon the agent selected. Reducing agents, as described above, can be advantageously used to maintain good shelf life of the formulation.

The compounds of Formula I or II can be in admixture with a suitable carrier, diluent, or excipient such as sterile water, physiological saline, glucose, or the like, and can contain auxiliary substances such as wetting or emulsifying agents, pH buffering agents, gelling or viscosity enhancing additives, preservatives, flavoring agents, colors, and the like, depending upon the route of administration and the preparation desired. See, *e.g*., "Remington: The Science and Practice of Pharmacy", Lippincott Williams & Wilkins; 20th edition (June 1, 2003) and "Remington's Pharmaceutical Sciences," Mack Pub. Co.; 18th and 19th editions (December 1985, and June 1990, respectively). Such preparations can include complexing agents, metal ions, polymeric compounds such as polyacetic acid, polyglycolic acid, hydrogels, dextran, and the like, liposomes, microemulsions, micelles, unilamellar or multilamellar vesicles, erythrocyte ghosts or spheroblasts. Suitable lipids for liposomal formulation include, without limitation, monoglycerides, diglycerides, sulfatides, lysolecithin, phospholipids, saponin, bile acids, and the like. The presence of such additional components can influence the physical state, solubility, stability, rate of *in vivo* release, and rate of *in vivo* clearance, and are thus chosen according to the intended application, such that the characteristics of the carrier are tailored to the selected route of administration.

For oral administration, the pharmaceutical compositions can be provided as a tablet, aqueous or oil suspension, dispersible powder or granule, emulsion, hard or soft capsule, syrup or elixir. Compositions intended for oral use can be prepared according to any method known in the art for the manufacture of pharmaceutical compositions and can include one or more of the following agents: sweeteners, flavoring agents, coloring agents and preservatives. Aqueous suspensions can contain the active ingredient in admixture with excipients suitable for the manufacture of aqueous suspensions.

Formulations for oral use can also be provided as hard gelatin capsules, wherein the active ingredient(s) are mixed with an inert solid diluent, such as calcium carbonate, calcium phosphate, or kaolin, or as soft gelatin capsules. In soft capsules, the active compounds can be dissolved or suspended in suitable liquids, such as water or an oil medium, such as peanut oil, olive oil, fatty oils, liquid paraffin, or liquid polyethylene glycols. Stabilizers and microspheres formulated for oral administration can also be used. Capsules can include push-fit capsules made of gelatin, as well as soft, sealed capsules made of gelatin and a plasticizer, such as glycerol or sorbitol. The push-fit capsules can contain the active ingredient in admixture with fillers such as lactose, binders such as starches, and/or lubricants such, as talc or magnesium stearate and, optionally, stabilizers. In instances where it is desirable to maintain a compound of Formula I or II in a reduced form (in the case of certain active metabolites), it can be desirable to include a reducing agent in the capsule or other dosage form.

Tablets can be uncoated or coated by known methods to delay disintegration and absorption in the gastrointestinal tract and thereby provide a sustained action over a longer period of time. For example, a time delay material such as glyceryl monostearate can be used. When administered in solid form, such as tablet form, the solid form typically comprises from about 0.001 wt. % or less to about 50 wt. % or more of active ingredient(s), preferably from about 0.005, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1 wt. % to about 2, 3, 4, 5, 6, 7, 8, 9, 10, 15, 20, 25, 30, 35, 40, or 45 wt. %.

Tablets can contain the active ingredients in admixture with non-toxic pharmaceutically acceptable excipients including inert materials. For example, a tablet can be prepared by compression or molding, optionally, with one or more additional ingredients. Compressed tablets can be prepared by compressing in a suitable machine the active ingredients in a free-flowing form such as powder or granules, optionally mixed with a binder, lubricant, inert diluent, surface active or dispersing agent. Molded tablets can be made by molding, in a suitable machine, a mixture of the powdered compound moistened with an inert liquid diluent.

Preferably, each tablet or capsule contains from about 10 mg or less to about 1,000 mg or more of a compound of the Formula I or II, more preferably from about 20, 30, 40, 50, 60, 70, 80, 90, or 100 mg to about 150, 200, 250, 300, 350, 400, 450, 500, 550, 600, 650, 700, 750, 800, or 900 mg. Most preferably, tablets or capsules are provided in a range of dosages to permit divided dosages to be administered. A dosage appropriate to the patient and the number of doses to be administered daily can thus be conveniently selected. In certain embodiments it can be preferred to incorporate two or more of the therapeutic agents to be administered into a single tablet or other dosage form (*e.g*., in a combination therapy); however, in other embodiments it can be preferred to provide the therapeutic agents in separate dosage forms.

Suitable inert materials include diluents, such as carbohydrates, mannitol, lactose, anhydrous lactose, cellulose, sucrose, modified dextrans, starch, and the like, or inorganic salts such as calcium triphosphate, calcium phosphate, sodium phosphate, calcium carbonate, sodium carbonate, magnesium carbonate, and sodium chloride. Disintegrants or granulating agents can be included in the formulation, for example, starches such as corn starch, alginic acid, sodium starch glycolate, Amberlite, sodium carboxymethylcellulose, ultramylopectin, sodium alginate, gelatin, orange peel, acid carboxymethyl cellulose, natural sponge and bentonite, insoluble cationic exchange resins, powdered gums such as agar, karaya or tragacanth, or alginic acid or salts thereof.

Binders can be used to form a hard tablet. Binders include materials from natural products such as acacia, tragacanth, starch and gelatin, methyl cellulose, ethyl cellulose, carboxymethyl cellulose, polyvinyl pyrrolidone, hydroxypropylmethyl cellulose, and the like.

Lubricants, such as stearic acid or magnesium or calcium salts thereof, polytetrafluoroethylene, liquid paraffin, vegetable oils and waxes, sodium lauryl sulfate, magnesium lauryl sulfate, polyethylene glycol, starch, talc, pyrogenic silica, hydrated silicoaluminate, and the like, can be included in tablet formulations.

Surfactants can also be employed, for example, anionic detergents such as sodium lauryl sulfate, dioctyl sodium sulfosuccinate and dioctyl sodium sulfonate, cationic such as benzalkonium chloride or benzethonium chloride, or nonionic detergents such as polyoxyethylene hydrogenated castor oil, glycerol monostearate, polysorbates, sucrose fatty acid ester, methyl cellulose, or carboxymethyl cellulose.

Controlled release formulations can be employed wherein the amifostine or analog(s) thereof is incorporated into an inert matrix that permits release by either diffusion or leaching mechanisms. Slowly degenerating matrices can also be incorporated into the formulation. Other delivery systems can include timed release, delayed release, or sustained release delivery systems.

Coatings can be used, for example, nonenteric materials such as methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and the polyethylene glycols, or enteric materials such as phthalic acid esters. Dyestuffs or pigments can be added for identification or to characterize different combinations of active compound doses

When administered orally in liquid form, a liquid carrier such as water, petroleum, oils of animal or plant origin such as peanut oil, mineral oil, soybean oil, or sesame oil, or synthetic oils can be added to the active ingredient(s). Physiological saline solution, dextrose, or other saccharide solution, or glycols such as ethylene glycol, propylene glycol, or polyethylene glycol are also suitable liquid carriers. The pharmaceutical compositions can also be in the form of oil-in-water emulsions. The oily phase can be a vegetable oil, such as olive or arachis oil, a mineral oil such as liquid paraffin, or a mixture thereof Suitable emulsifying agents include naturally-occurring gums such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsions can also contain sweetening and flavoring agents.

When a compound of the Formula I or II is administered by intravenous, parenteral, or other injection, it is preferably in the form of a pyrogen-free, parenterally acceptable aqueous solution or oleaginous suspension. Suspensions can be formulated according to methods well known in the art using suitable dispersing or wetting agents and suspending agents. The preparation of acceptable aqueous solutions with suitable pH, isotonicity, stability, and the like, is within the skill in the art. A preferred pharmaceutical composition for injection preferably contains an isotonic vehicle such as 1,3-butanediol, water, isotonic sodium chloride solution, Ringer's solution, dextrose solution, dextrose and sodium chloride solution, lactated Ringer's solution, or other vehicles as are known in the art. In addition, sterile fixed oils can be employed conventionally as a solvent or suspending medium. For this purpose, any bland fixed oil can be employed including synthetic mono or diglycerides. In addition, fatty acids such as oleic acid can likewise be used in the formation of injectable preparations. The pharmaceutical compositions can also contain stabilizers, preservatives, buffers, antioxidants, or other additives known to those of skill in the art.

The duration of the injection can be adjusted depending upon various factors, and can comprise a single injection administered over the course of a few seconds or less, to 0.5, 0.1, 0.25, 0.5, 0.75, 1, 2, 3, 4, 5, 6, 7, 8, 9, 10, 11, 12, 13, 14, 15, 16, 17, 18, 19, 20, 21, 22, 23, or 24 hours or more of continuous intravenous administration.

The anti-HIV and anti-AIDS compositions comprising a compound of Formula I or II can additionally employ adjunct components conventionally found in pharmaceutical compositions in their art-established fashion and at their art-established levels. Thus, for example, the compositions can contain additional compatible pharmaceutically active materials for combination therapy (such as supplementary antimicrobials, antipruritics, astringents, local anesthetics, anti-inflammatory agents, reducing agents, and the like), or can contain materials useful in physically formulating various dosage forms of the preferred embodiments, such as excipients, dyes, thickening agents, stabilizers, preservatives or antioxidants.

The compounds of Formula I or II can be provided to an administering physician or other health care professional in the form of a kit. The kit is a package which houses a container which contains the compound(s) in a suitable pharmaceutical composition, and instructions for administering the pharmaceutical composition to a subject. The kit can optionally also contain one or more additional therapeutic agents, e.g., zidovudine. For example, a kit containing one or more compositions comprising compound(s) of Formula I or II in combination with one or more additional antiretroviral, antibacterial, and/or anti-infective agents can be provided, or separate pharmaceutical compositions containing a compound of the preferred embodiments and additional therapeutic agents can be provided. The kit can also contain separate doses of a compound of Formula I or II for serial or sequential administration. The kit can optionally contain one or more diagnostic tools and instructions for use. The kit can contain suitable delivery devices, e.g., syringes, and the like, along with instructions for administering the compound(s) and any other therapeutic agent. The kit can optionally contain instructions for storage, reconstitution (if applicable), and administration of any or all therapeutic agents included. The kits can include a plurality of containers reflecting the number of administrations to be given to a subject. A kit for the treatment of HIV or AIDS is disclosed that includes amifostine or another compound of a Formula I or II and one or more antiretroviral agents currently employed for the treatment HIV. For example, reverse transcriptase inhibitors such as zidovudine, didanosine, zalcitabine, stavudine, 3TC, and nevirapine; protease inhibitors; cytokines; immunomodulators, and antiinfectives commonly employed to combat AIDS-related infections can be employed.

The compounds of Formula I or II can be administered prophylactically for the prevention of HIV or AIDS. Alternatively, therapy is preferably initiated as early as possible following the onset of signs and symptoms of an HIV infection, or following exposure to HIV. The administration route, amount administered, and frequency of administration will vary depending on the age of the patient, the severity of the infection, and any associated conditions. Contemplated amounts, dosages, and routes of administration for the compounds of Formula I or II for treatment of HIV infections are similar to those established for conventional antiretroviral agents. Detailed information relating to administration and dosages of conventional antiretroviral agents can be found in the Physician's Desk Reference, 47th edition. This information can be adapted in designing treatment regimes utilizing the compounds of Formula I or II. It is expected that that anti-HIV therapies combining an aminothiol with a nucleoside analog will have an additive or synergistic activity, making it possible to reduce the doses of the drugs currently used to treat HIV infections (Bergamini et al. (1996), J Infect Dis 174(1): 214-8). The combination of an aminothiol with a nucleoside analog is expected to simultaneously provide improved anti-HIV therapy and protection against NRTI-induced side effects, especially those associated with nuclear DNA or mitochondrial DNA damage.

While it is generally preferred to administer the compounds of Formula I or II at the dosages described above, in certain other embodiments it can be desirable to administer lower or higher dosages, depending upon various factors (the specific compound of the Formula I or II employed, the patient to be treated, and the like). For example, contemplated amounts of the compounds of the Formula I or II for oral administration to treat HIV infections can be from as low as about 10 mg or less to as high as about 2000 mg or more administered from about every 4 hours or less to about every 6, 12, or 24 hours or more for about 5 days or less to about 10 days or more or until there is a significant improvement in the condition. For suppressive therapy to inhibit the onset of infection in susceptible individuals, doses of from about 10 mg or less to about 1000 mg or more can be orally administered once, twice, or multiple times a day, typically for up to about 12 months, or, in certain circumstances, indefinitely (from about 10 mg/day to about 1,000 mg/day). When treatment is long term, it can be desirable to vary the dosage, employing a higher dosage early in the treatment, and a lower dosage later in the treatment.

The single highest dose of amifostine administered to an adult human as documented in the literature was 1330 mg/m². Children have been administered single doses of amifostine of up to 2700 mg/m² with no untoward effects. The literature indicates that multiple doses (up to three times the recommended single dose of 740 to 910 mg/m²) have been safely administered within a 24-hour period. Repeated administration of amifostine at two and four hours after the initial dose does not appear to result in an increase in side effects, especially nausea, vomiting, or hypotension. It appears that the most significant deleterious side effect from the administration of amifostine is hypotension.

Contemplated amounts of the compounds of the Formula I or II , methods of administration, and treatment schedules for individuals with AIDS are generally similar to those described above for treatment of HIV.

Known side effects of amifostine include decrease in systolic blood pressure, nausea, and vomiting. If such side effects are observed for the particular thiophosphate administered, it is generally preferred to administer an antiemetic medication prior to, or in conjunction with the thiophosphate. Suitable antiemetic medications include antihistamines (*e.g.*, buclizine, cyclizine, dimenhydrinate, diphenhydramine, meclizine), anticholinergic agents (*e.g.*, scopolamine), dopamine antagonists (*e.g.*, chlorpromazine, droperidol, metoclopramide, prochlorperazine, promethazine), serotonin antagonists (*e.g.*, dolasetron, granisetron, ondansetron), or other agents (*e.g.*, dexamethasone, methylprednisole, trimethobenzamide).

When the compounds of Formula I or II are to be administered for their effectiveness as cytoprotective agents when employed in combination with a nucleoside reverse transcriptase inhibitor, e.g., zidovudine, it is generally preferred that a lower amount of the compound be administered. A unit dosage form comprising from about 100 mg to about 300 mg of the compound can typically provide a cytoprotective effect. Alternatively, higher dosages can be administered to increase antiretroviral effectiveness. The NRTI can be administered at conventional dosage levels e.g., 600 mg/day for zidovudine; however, because the compounds of Formula I or II possess antiretroviral activity, the amount of NRTI administered can be desirably be reduced while still achieving a satisfactory antiretroviral effect, e.g., by administering a dosage of the NRTI that is half or less of the dosage that is conventionally administered. For example, when zidovudine is administered, 300 mg/day can be administered, more preferably 100 to 200 mg/day, in combination with a compound of Formula I or II at a dosage as described elsewhere herein. By administering the compounds of Formula I or II in combination with at least one NRTI, antiretroviral effectiveness characteristic of that achievable by the NRTI at conventional dosages alone is obtained, but with a dramatically decreased adverse cytotoxic effect that would be observed for the NRTI alone at conventional dosages.

### Reference Experiments

The purity of the WR-1065 used in the experiments described below is unknown. WR-1065 is sensitive to oxidation (and possibly other reactions) and can undergo reaction to forms that appear to lack antiretroviral activity. Accordingly, in the tables below and in the preferred dosages provided above, the indicated concentrations represent the maximum amount of active compound that could be present; the true concentration of active compound is less than that indicated but cannot be determined definitively. In addition, for concentrations of WR-1065 of less than 100 µM, estimates of the true efficacy of the compound are limited by the fact that the compound has been found to be inactivated by a variety of medium components (Grdina et al. (2000), Drug Metabol Drug Interact 16(4): 237-79.). Below approximately 50 µM, this problem becomes especially severe.

### Antiretroviral Effects of AZT in the Presence of WR-1065 in Human Cells

Experiments were conducted to determine the antiretroviral effects of AZT in the presence of WR-1065, the active metabolite of amifostine. Peripheral blood mononuclear cells (PBMCs) obtained from the NIH Blood Bank were cultured with phytohemagglutinin (PHA) for 48 hours to create PHA blasts. The cells were cultured in RPMI-1640 medium with 10% fetal bovine serum (FBS), 1% penicillin/streptomycin and glutamine, and 10% Interleukin-2 (IL-2). The PHA blasts were then infected with HIV for two hours (see Perno et al. (1988), J. Exptl. Med. 168:111; Aquaro et al. (1988), J. Medical Virology 68:479-488).

Noting the caveats outlined above regarding purity of the WR-1065 used, WR-1065 (from the NCI Chemical Carcinogen Repository; see Hoffman et al. (2001), Env. Mol. Mut. 37:117) was weighed (Mol. Wt. 134) and stored frozen in RPMI-1640 medium as a 10 mg/ml solution (13.4 µL of the solution added to 1 ml of RPMI-1640 yields 1000 µM solution). AZT (SIGMA, Mol. Wt. 267) was dissolved in phosphate-buffered saline (PBS) at a concentration of 36 mM (9.72 mg/ml), 0.01 ml (97 µg) of the resulting solution was added to 3.6 ml RPMI-1640 medium to yield a 26.9 µg/ml solution, and 0.1 ml (2.69 µg) of that resulting solution was added to 1 ml of RPMI-1640 medium to yield a 10 µM solution of AZT. The HIV-infected PHA blasts were incubated with 10 µM AZT in the presence or absence of 1000 µM WR-1065 prepared as described above.

At 72 hours, HIV infection status was monitored in five experimental groups by measuring p24 using an ELISA kit (RETRO-TEK HIV-1, p24 Extended Range ELISA, ZMC catalog # 0801137). The HIV infection status for the five experimental groups is provided in Table 5 (see Experiment #1 data).

**Table 5.**

| Antiretroviral Efficacy of Zidovudine (AZT) Versus WR-1065 | | | | |
|---|---|---|---|---|
| **Treatment Group** | **10 µM AZT** | **WR-1065** | **HIV Viral P24 pg/ml** | **Estimated percent inhibition** |
| *Experiment #1* | | | | |
| PHA blasts | No | No | 0.28 | N. A. |
| PHA blasts + HIV | No | No | 498.62 | 0% |
| PHA blasts + HIV | Yes | No | 0.13 | ∼ 100% |
| PHA blasts + HIV | Yes | Yes (1000 µM) | 0.13 | ∼ 100% |
| PHA blasts + HIV | No | Yes (1000 µM) | 0.13 | ∼ 100% |

| *Experiment #2* | | | | |
|---|---|---|---|---|
| PHA blasts | No | No | 8.99 | N.A. |
| PHA blasts + HIV | No | No | 176.45 | 0% |
| PHA blasts + HIV | Yes | No | 9.07 | 94.9% |
| PHA blasts + HIV | No | Yes (1000 µM) | 8.92 | 94.9% |
| PHA blasts + HIV | No | Yes (330 µM) | 9.31 | 94.7% |
| PHA blasts + HIV | No | Yes (100 µM) | 8.92 | 94.9% |

| *Experiment #3* | | | | |
|---|---|---|---|---|
| PHA blasts | No | No | 3.0 | N.A. |
| PHA blasts + HIV | No | No | 44,455.8 | 0% |
| PHA blasts + HIV-1 | Yes | No | 2.9 | ∼ 100% |
| PHA blasts + HIV-1 | No | Yes (66 µM) | 33.2 | 99.9% |
| PHA blasts+ HIV-1 | No | Yes (33 µM) | 503.2 | 98.9% |
| PHA blasts + HIV-1 | No | Yes (10 µM) | 911.2 | 98.0% |
| PHA blasts + HIV-1 | No | Yes (5 µM) | 10,293.7 | 76.8% |

The test results from Experiment #1 (Table 5) demonstrated that 1000 µM WR-1065 inhibits HIV completely in human PBMCs and does not appear to alter the effect of 10 µM AZT.

A second experiment was conducted to determine the antiretroviral effects of lower doses of WR-1065. Tests were conducted on PHA blasts cultured as described for the previous experiments. The PHA blasts were infected with HIV for two hours, then incubated with either 10 µM AZT (see Experiment #1), or 100 µM, 330 µM, or 1000 µM WR-1065 (see Experiment #1). The AZT solution was prepared as described for the previous experiments. WR-1065 was prepared from a 10 mg/ml solution in RPMI-1640 medium (1.34, 4.0, or 13.4 µL of the solution was added to 1 ml of RPMI-1640 to yield a 100, 330, or 1000 µM solution, respectively). At 72 hours, HIV infection status was monitored in six experimental groups by measuring p24 using an ELISA kit as described for the previous experiments. The HIV infection status for the six experimental groups is provided in Table 5 (see Experiment #2 data).

The test results from Experiment #2 (Table 5) demonstrated that concentrations of WR-1065 as low as 100 µM inhibit HIV infection completely in human PBMCs.

A third experiment was conducted to determine the lowest doses of WR-1065 that inhibit HIV. Tests were conducted on PHA blasts cultured as described for the previous experiments. The PHA blasts were then infected with HIV for two hours, then incubated with either 10 µM AZT (see Experiment #1) alone, or 66 µm, 33 µM, 10 µm, or 5 µM WR-1065 (see Experiment #1). The AZT solution was prepared as described for the previous experiments. WR-1065 (10 mg/ml) was diluted 1:100 in RPMI-1640 medium, then 88, 44, 13.4, or 6.7 µL of the solution was added to 1 ml of RPMI-1640 to yield a 66, 33, 10, or 5 µM solution, respectively. At 72 hours, HIV infection status was monitored in six experimental groups by measuring p24 using an ELISA kit as described for the previous experiments. The HIV infection status for the seven experimental groups is provided in Table 5 (see Experiment #3 data).

The test results for Experiment #3 (Table 5) demonstrated that WR-1065 is a highly effective antiretroviral agent at concentrations similar to those used for AZT (*i.e.,* 10 µM).

### Viability of Human Cells in the Presence of WR-1065

Experiments were conducted to determine the viability of cells exposed to various concentrations of WR-1065. Tests were conducted on PHA blasts cultured as described for the previous experiments, but were not incubated in the presence of HIV. WR-1065 was prepared from a 10 mg/ml solution in RPMI-1640 medium and diluted to the desired concentrations. Cell viability was measured 72 hours after exposure to WR-1065. Percentage of viable cells (compared to unexposed controls) is provided in Table 6.

**Table 6.**

| WR-1065 Concentration (µM) | % Viable Cells (compared to control) |
|---|---|
| 1000 | 30 |
| 500 | 73 |
| 100 | 63 |
| 50 | 89 |
| 10 | 90 |
| 5 | 82 |
| 1 | 83 |

The test results demonstrated acceptable cell viability for all concentrations tested, and particularly good cell viability for concentrations of 50 µM and lower.

### Comparison of Antiretroviral Activity of Cysteamine and WR-1065

Cysteamine (chemical formula H₂NCH₂CH₂SH) has been demonstrated to have anti-HIV activity (Ho et al. (1995), AIDS Res Hum Retroviruses 11(4): 451-9; Bergamini et al. (1996), J Infect Dis 174(1): 214-8). Using an *in vitro* assay system as described above, 200 µM cysteamine effectively suppressed (∼100%) HIV replication. In comparison, WR-1065 effectively suppressed (>99.9%) HIV replication at a concentration of less than 100 µM. Thus, the *in vitro* anti-HIV activity of WR-1065 appears to be 2-fold more potent than that of cysteamine. In addition, the duration of action of cysteamine was determined to be very short; to achieve an anti-HIV effect, fresh cysteamine had to be added to the cell culture system every 12 hours. WR-1065, however, had a long duration of action; the HIV inhibition was present even if it was added to the culture system only once in a 72 hour period. These findings support the hypothesis that cysteamine and WR1065 may exert their antiretroviral effects via different mechanisms.

### Comparison of Antiretroviral Activity of Cystamine and WR-1065

Cystamine (chemical formula H₂N(CH₂)₂SS(CH₂)₂NH₂), the oxidized form of cysteamine, has been demonstrated to have DNA binding capacity, radioprotective capacity, and the ability to shift the equilibrium of DNA from the A-form towards the B-form (Allegra et al. (2002), Amino Acids 22(2): 155-66). WR-1065, the active form of WR-2721, has also been shown to bind to DNA in the minor groove, and also to shift the B/A-DNA equilibrium towards the B-form. Accordingly, it can be inferred that cystamine and its reduced form cysteamine are capable of binding to other nucleic acids in addition to DNA, and have some ability to bind to and/or to interact with proteins. In addition, it is possible that WR-33278 (the disulfide of WR-1065) may also display some of the same nucleic acid and protein affinities described for cystamine and for WR-1065.

A comparison of the DNA phosphate binding capacity of cystamine versus WR-1065 demonstrated that these two compounds have similar binding affinities under similar *in vitro* conditions. (Smoluk et al. (1986), Radiat Res. 107(2):194-204). However, WR-1065 is a larger molecule than cysteamine because it contains an additional moiety: -(CH₂)₃NH₂. Thus, it can be hypothesized that WR-1065 can bind to and thereby block a larger fragment of a nucleic acid than cysteamine. Similar hypotheses can be formulated for WR-33278, the disulfide of WR-1065. It can further be hypothesized that the differential binding characteristic of WR-1065 versus cysteamine may be responsible for WR-1065's improved antiretroviral efficacy. It is possible that WR-1065's antiretroviral activity is due in part to its ability to bind to and to block critical sites on nucleic acids and/or proteins. (Allegra et al. (2002), Amino Acids 22(2): 155-66; North et al., (2002), Mol. Carcinog. 33(3): 181-8)_{.} Blockage of nucleic acid sites and/or proteins is considered to be a possible mechanism for the aminothiols' modulation of enzyme function *(*Brekken et al. (198b), J Biol Chem 273(41): 26317-22).

As further evidence of the potential importance of WR-1065's nucleic acid binding capacity to its antiretroviral activity, it has been demonstrated that WR-1065 has antiviral efficacy against three different species of adenovirus and two different strains of influenza. These are dramatically different types of viruses with significantly different modes of replication. The only currently recognized, common replication element shared by all of these viruses, as well as HIV, is a requirement for single-stranded or double-stranded RNA during part of the replication cycle. Thus, the demonstration of broad-spectrum antiviral activity supports the hypothesis that the ability of the antiretroviral agent WR-1065 to bind to RNA plays a role in the observed antiretroviral effect.

### Experiments - Mitigation of Genotoxic Effects of NRTI Therapy

Experiments were conducted to determine whether amifostine and WR-1065 can mitigate the genotoxic effects of NRTI therapy, specifically, aidovudine (AZT)-induced mutagenesis, and to ascertain whether or not NRTI antiretroviral efficacy is altered in the presence of such drugs.

Human peripheral blood mononuclear cells (PBMCs) were infected with HIV-1 to determine dose-response for inhibition of virus replication by AZT; dose-responses for amifostine and WR1065; and whether or not the combination of AZT plus an aminothiol altered antiretroviral capacity compared to AZT alone.

Human peripheral blood mononuclear cells (PBMCs) discarded from blood bank material were cultured in the presence of phytohemaglutinin (PHA) for 48 hr to produce PHA blasts. PHA-blasts were grown in microtiter plate wells (50,000 cells/well) and infected for 2 hr with HIV-1_{BZ-167}. AZT, WR-1065, and amifostine with alkaline phosphatase were added separately or in various combinations after 2 hrs of infection, and the cells were incubated an additional 72 hr. Cell survival (cytotoxicity) was determined by trypan blue. HIV-1 growth/replication was determined by p24 ELISA kit (either the HIV.-1 p24 Extended Range ELISA from ZMC or the HIV-1 p24 Antigen Capture Assay Kit from NCI-Frederick).

AZT concentrations of 0.8, 3.0, and 5.0 µM inhibited virus replication by 85-96%, and the same AZT doses combined with 5-26 µM WR-1065 inhibited virus replication by 91-97%. WR-1065, at 26 µM, did not inhibit the antiretroviral activity of 0.8-3.0 µM AZT. A dose-response for WR-1065 (2.5-103.0 µM) showed 50% Inhibition of virus replication at 3.1 ± 0.6 µM (mean ± SE, n=3). A dose-response for AZT (1.9-117.0 nM) showed 50% Inhibition was at 3.5 ± 0.7 ηM (mean ± SE, n=3). When tested alone, AZT at 7.75 µM inhibited virus replication by 96.7±5.1% (mean ±SD, n=4) with 54±15% target cell survival. Equimolar concentrations of amifostine and WR-1065 showed similar inhibition of HIV-1 replication. Amifostine at 50 µM inhibited virus replication by 75.4 ± 8.3% (mean ± SE, n=4) and cell survival was 53 ± 10% (mean ± SE, n=4). Similarly, WR-1065 concentrations of 50 and 100 µM blocked virus replication by 83.2±18.6% (n=5) and 92.0±11.0% (n=5), respectively, with cell survival at 55±22% and 42±18%, respectively. WR-1065 concentrations of 26, 52 and 103 µM blocked virus replication by 65.0 ± 7.5% (mean ± SE, n=5), 88.7 ± 5.5% (n=7) and 93.9 ± 3.1 % (n=8), respectively, with cell survival at 37%, 49% and 78%, respectively. The HIV-1 growth inhibition at a dose of 103-206 µM of WR-1065 was ≥94%, and that concentration is similar to human plasma levels reached after administration of the recommended dose of amifostine. The dose of AZT providing a 50% reduction in HIV-1 replication is about 1000-fold lower than the dose of WR-1065 providing a 50% reduction in HIV-1 replication.

Some of the cytotoxicity observed in the experiments may have been due to the formation of toxic bi-products of WR-1065 by Cu-dependent amine-oxidases found in serum. Aminoguanidine has been previously shown to block the action of these enzymes and to have very low cytotoxicity. In TK6 cells (human lymphoblastoid cells heterozygous for the enzyme thymidine kinase) the 14-day survival in the continuous presence of 50-100 µM WR-1065 was <20%, however if 10 mg/ml aminoguanidine was added from day 4, the 14-day survival was 70-80%. Therefore, cell survival in the PBMCs might have been higher if aminoguanidine had been added at the beginning of the treatment period.

Kalebic and Schein [(1994) AIDS Res Hum Retroviruses. 106, 727-33] hypothesized that the anti-HIV effects they found in the presence of *milli*-molar concentrations of aminothiols were the result of inhibition of cytokine production. In the above experiments using *micro*-molar concentrations of aminothiols to inhibit HIV-1-replication, it is not likely that this is the major mechanism of action. Because Grdina and colleagues [Woloschak et al. (1995) Cancer Res. 5521, 4788-92] showed different modes of action for WR-1065 at low (micromolar) and high (millimolar) drug concentrations, it is likely that the inhibition of viral replication that we are seeing is not due to an inhibition of cytokine production, but is instead due to some other mechanism.

The data show that amifostine and WR-1065 do not compromise the antiretroviral efficacy of AZT. When used alone, these aminothiols also substantially inhibit HIV-1 replication. The data demonstrate that aminothiols, especially aminothiols in micromolar concentrations, have the potential to be used as adjunct therapy for the treatment of HIV.-1. The use of amifostine along with HAART can allow reduction in NRTI dose levels, and may be useful in patients who have developed resistance to NRTI therapy.

It can also be desirable to administer aminoguanidine, or another compound that blocks the activity of Cu-dependent amine-oxidases, in combination with WR-1065, so as to minimize cytotoxic effects of WR-1065.

The p24 assay that was used to evaluate inhibition of viral replication may underestimate the inhibitory effects of amifostine and WR-1065 because this assay only reflects events occurring before the synthesis of p24 in the HIV-1 life cycle. Another structurally similar aminothiol (cystamine) blocks viral replication early and at a later step occurring after p24 synthesis. Therefore, WR-1065 may have similar activity, and the later stage inhibition would not be reflected in the assay. Difficulty in measuring combined cytotoxicity of AZT plus WR-1065 is consistent with a complicated series of events contributing to HIV-1 replication inhibition.

### Experiments - Effect of WR-1065 on HIV Replication

Experiments were conducted using human T-lymphocytes to determine the *intra*-cellular concentration of WR-1065 necessary to reduce HIV replication by - 100% percent. The concentration was determined to be - 50 picornoles/10⁶ cells. The inter-cellular concentration, expressed on an individual cell basis, is approximately 1000-fold lower and in the range of that determined for AZT. Calabro-Jones et al. [(1998) Radiat Res. 149, 550-559] showed significant WR-1065 cytotoxicity at *inter*-cellular levels of -30 nanomoles per cell. Thus, amifostine has efficacy inside of cells at concentrations that are several orders of magnitude below those associated with cytotoxicity.

### Discussion

While not wishing to be bound by any particular theory, it is believed that WR-1065 functions as an antiretroviral agent because its structure renders it bifunctional, combining two distinct properties. First, a portion of the compound is believed to be responsible for binding to nucleic acids (DNA, RNA), and possibly also to some proteins. For WR-1065, this property is believed to be largely attributable to the following moiety: -(CH₂)₃NH₂. A second portion of WR-1065 is believed to be largely responsible for the antiretroviral effects that have been observed; this portion of the compound has the following structure: -NH(CH₂)₂SH. It is believed that the sulfhydryl group needs to be in the reduced state in order for maximal antiretroviral effects to be observed for the compound; however, it is possible that the sulfhydryl group may remain functional if oxidized to the disulfide (as in WR-33278). Both of these components contribute to maximizing antiretroviral effects of the compounds of Formula I or II (*e.g.*, both the -(CH₂)₃NH₂ and the -NH(CH₂)₂SH moieties of amifostine). It is hypothesized that the first portion of the molecule functions to align the molecule in close proximity to critical binding sites on nucleic acids and/or proteins, and the second portion of the molecule functions in a reaction that contributes to antiretroviral efficacy.

Variations in the chemical structure of the moiety -(CH₂)₃NH₂ may be tolerated without losing or altering the antiretroviral efficacy of the compound, so long as the resulting structure retains a binding capacity/affinity that is similar to that observed with -(CH₂)₃NH₂ itself. Such variations may include more or less than three carbon atoms in the alkyl group, and a branched alkyl chain, or lower alkyl substituents on the amino group. The potential importance of the moiety -(CH₂)₃NH₂ (or suitable variant) to antiretroviral effectiveness of the compounds of Formula I or II is supported by the work of Laayoun *et al.*, who demonstrated a significant increase in anti-mutagenic activity when cysteamine or WR-2721 was tethered to a chromophore (quinoline or acridine) that increased DNA binding (Laayoun et al. (1994), Int J Radial Biol 66(3): 259-66). It is hypothesized that the antimutagenic activity of the aminothiols may result from a mode of action that is also relevant for their antiretroviral activity, suggesting that retroviral affinity could be altered by changing the chemical structure of the moiety-(CH₂)₃NH₂.

*Gutschow et al.* described two compounds that are demonstrated to display significant antiviral activity (Gutschow et al. (1995), Pharmazie 50(10): 672-5.). These compounds differ in structure considerably from the compounds of Formula I or II , but the two most efficacious compounds have, as a portion of their structure, a cysteamine-like group. Gutschow *et al.* tested a number of compounds, some of which differed in the number of carbon atoms separating the sulfhydryl group and its adjacent amino group, and noted that the optimal antiviral effect was observed using molecules that had a cysteamine-like group and that this effect was diminished if the number of -CH₂- groups separating the sulfhydryl group and the first amino group was increased to three, as occurs in the aminothiols WR-151327 (chemical formula CH₃NH(CH₂)₃NH(CH₂)₃SPO₃H₂) and WR-151326 (chemical formula CH₃NH(CH₂)₃NH(CH₂)₃SH). Accordingly, altering the structure of the cysteamine-like group to include three -CH₂- groups between the sulfhydryl group and the amino group significantly reduced the capacity of the compound to function as an antiviral agent.

In contrast to WR-151327 and WR-151326, the compounds of Formula I or II have only two -CH₂- groups separating the sulfhydryl group from the first amino group, and thus possess superior antiretroviral properties. The antiretroviral activity of WR-1065 is significantly greater than that reported for WR-151326 (see Kalebic *et al.,* (1994)), and thus WR-1065 has functional capacities that are different from those of WR-151327 and WR-151326.

References: Methods and materials Allegra et al. (2002). "The ability of cystamine to bind DNA." Amino Acids 22(2): 155-66; Bergamini et al. (1996). "In vitro inhibition of the replication of human immunodeficiency virus type 1 by beta-mercaptoethylamine (cysteamine)." J Infect Dis 174(1): 214-8; Brekken et al. (1998). "Trypanosoma brucei gamma-glutamylcysteine synthetase. Characterization of the kinetic mechanism and the role of Cys-319 in cystamine inactivation." J Biol Chem 273(41): 26317-22; Clark et al. (1997). "The aminothiol WR-1065 protects T-lymphocytes from ionizing radiation-induced deletions of the HPRT gene." Cancer Epidemiol. Biomarkers and Prevention 6:1033; Grdina et al. (2000). "Amifostine: mechanisms of action underlying cytoprotection and chemoprevention." Drug Metabol Drug Interact 16(4): 237-79; Gutschow et al. (1995). "[Bis((2,4-dioxo-1,2,3,4-tetrahydroquinaaolin-3-yl)alkyl)disulfane and 3-(mercaptoalkyl)quinazolin-2,4(1H,3H)-dione: synthesis by ring transformation and antiviral activity. 42. Heterocyclic azines with heteroatoms in the 1- and 3- positions]." Pharmazie 50(10): 672-5; Hamasaki et al. (2001). "Aminoglycoside antibiotics, neamine and its derivatives as potent inhibitors for the RNA-protein interactions derived from HIV-1 activators." Bioorg Med Chem Lett 11(4):591-4; Ho et al. (1995). "Cystamine inhibits HIV type 1 replication in cells of monocyte/macrophage and T cell lineages." AIDS Res Hum Retroviruses 11(4): 451-9; Kalebic et al. (1994). "Organic thiophosphate WR-151327 suppresses expression of HIV in chronically-infected cells." AIDS Research and Human Retroviruses 10:727; Laayoun et al. (1994). "Aminothiols linked to quinoline and acridine chromophores efficiently decrease 7,8-dihydro-8-oxo-2'-deoxyguanosine formation in gamma-irradiated DNA." Int J Radiat Biol 66(3): 259-66; Lacourciere et al. (2000). "Mechanism of neomycin and Rev peptide binding to the Rev responsive element of HIV.-1 as determined by fluorescence and NMR spectroscopy." Biochemistry 39(19):5630-41; Li et al. (2001). "A heterocyclic inhibitor of the REV-RRE complex binds to RRE as a dimer." Biochemistry 40(5):1150-8; List et al. (1997). "Stimulation of hematopoiesis by amifostine in patients with myelodysplastic syndrome." Blood 90(9): 3364-9; Luedtke et al. (2003). "Fluorescence-based methods for evaluating the RNA affinity and specificity of HIV-1 Rev-RRE inhibitors." Biopolymers 70(1):103-19; Newton et al. (1996). "Transport of aminothiol radioprotectors into mammalian cells: passive diffusion versus mediated uptake." Radiat Res 146(2):206-15; Nguyen et al. (2003). "Amifostine and curative intent chemoradiation for compromised cancer patients." Anticancer Research 23:1649; Nishizono et al. (2000). "Synthesis of biomimetic analogs of neomycin B: potential inhibitors of the HIV RNA Rev response element." Nucleosides Nucleotides Nucleic Acids 19(1-2):283-95.; North et al. (2002). "Restoration of wild-type conformation and activity of a temperature sensitive mutant of p53 (p53(V272M)) by the cytoprotective aminothiols WR1065 in the esophageal cancer cell line TE-1." Mol. Carcinog. 33(3): 181-8; Oiry et al. (2004). "Synthesis and biological evaluation in human monocyte-derived macrophages of N-(N-acetyl-L-cysteinyl)-S-acetylcysteamine analogues with potent antioxidant and anti-HIV activities." J Med Chem 47(7): 1789-95; Olsen et al. (1990). "Interaction of the human immunodeficiency virus type 1 Rev protein with a structured region in env mRNA is dependent on multimer formation mediated through a basic stretch of amino acids." Genes Dev 4(8): 1357-64; Perno et al. (1988). "Inhibition of HIF-1 replication in fresh and cultured human peripheral blood monocytes by azidothymidine." J Experimental Medicine 168:1111*;* Qian-Cutrone et al. (1996). "Niruriside, a new HIV. REV/RRE binding inhibitor from Phyllanthus niruri." J Nat Prod 59(2):196-9; Reddy et al. (1999). "Inhibition of HIV replication by dominant negative mutants of Sam68, a functional homolog of HIV-1." Rev Nat Med 5(6):635-42; Rossio et al. (1998). "Inactivation of HIV Type I infectivity with preservation of conformational and functional integrity of virion surface proteins." Journal of Virology 72:7992; Santini et al. (1999). "The potential of amifostine: from cytoprotectant to therapeutic agent." Haematologica 84(11): 1035-42; Schuchter, L. M. (1996). "Guidelines for the administration of amifostine." Semin Oncol 23(4 Suppl 8): 40-3; U.S. Pat. No. 5,824,664 to Schein et al. "Suppression of HIV Expression by Organic Thiophosphate."; Xiao et al. (2001). "Inhibition of the HIV-1 rev-RRE complex formation by unfused aromatic cations." Bioorg Med Chem 9(5):1097-113; Zang et al. (1999). "Distinct export pathway utilized by the hepatitis B virus posttranscriptional regulatory element." Virology 259(2):299-304; Zapp et al. (1997). "Modulation of the Rev-RRE interaction by aromatic heterocyclic compounds." Bioorg Med Chem 5(6):1149-55.

The term "comprising" as used herein is synonymous with "including," "containing," or "characterized by," and is inclusive or open-ended and does not exclude additional, unrecited elements or method steps.

All numbers expressing quantities of ingredients, reaction conditions, and so forth used in the specification are to be understood as being modified in all instances by the term "about." Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

This invention is susceptible to modifications, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the scope of the claims.

## Claims

1. Use of a compound of Formula (II) or a pharmaceutically acceptable salt or solvate thereof in the preparation of a medicament for treating or preventing a human immunodeficiency virus infection wherein Formula (II) has the structure: 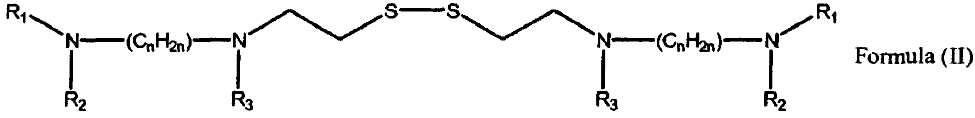 wherein each of R₁, R₂ and R₃ is independently selected from hydrogen and C₁₋₆ alkyl, and wherein n is an integer from 1 to 10.

2. The use of claim 1, wherein said medicament is employed in combination with at least one nucleoside reverse transcriptase inhibitor.

3. The use of claim 1 or 2, wherein the compound is

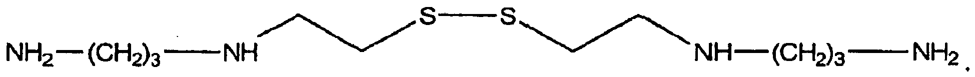

4. The use of claim 1 or 2, wherein the compound is

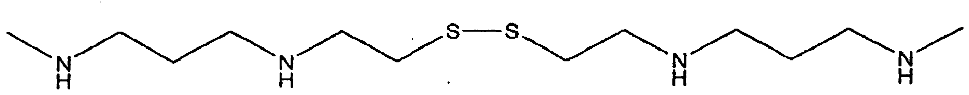

5. The use of claim 2, wherein the nucleoside reverse transcriptase inhibitors are selected from the group consisting of zidovudine, didanosine, stavudine, zalcitabine, lamivudine, abacavir, and combinations thereof.

6. The use of claim 5, wherein at least one of the nucleoside reverse transcriptase inhibitors is zidovudine.

7. The use of claim 6, wherein the zidovudine is administered to the patient at a daily dosage of from 300 mg/day to 400 mg/day.

8. The use of claim 1 or claim 2, wherein the medicament comprises from 500 mg to 700 mg of the compound.

9. A compound of Formula (II) or a pharmaceutically acceptable salt or solvate thereof for use in treating or preventing a human immunodeficiency virus infection wherein Formula (II) has the structure: 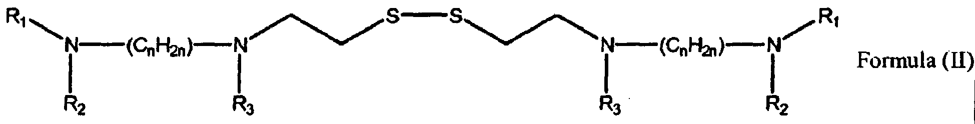 wherein each of R₁, R₂ and R₃ is independently selected from hydrogen and C₁₋₆ alkyl, and wherein n is an integer of from 1 to 10.

10. The compound for use according to claim 9, wherein the compound is

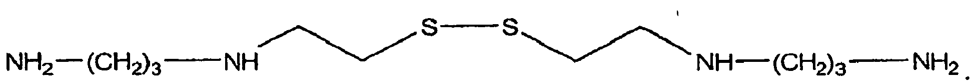

11. The compound for use according to claim 9, wherein the compound is

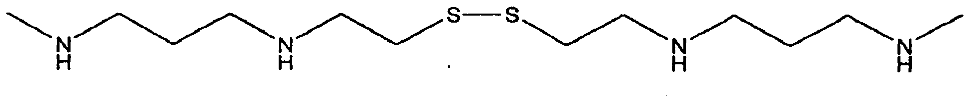

## Patentansprüche

1. Verwendung einer Verbindung der Formel (II) oder eines pharmazeutisch annehmbaren Salzes oder Solvats davon zur Herstellung eines Medikaments zur Behandlung oder Prävention einer HIV-Infektion, wobei die Formel (II) die Struktur 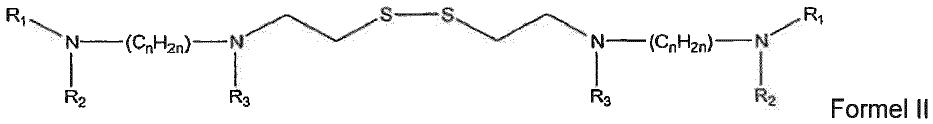 worin jedes von R₁, R₂ und R₃ unabhängig ausgewählt ist aus Wasserstoff und C₁₋₆-Alkyl und worin n eine ganze Zahl von 1 bis 10 ist,
aufweist.

2. Verwendung nach Anspruch 1, wobei das Medikament verwendet wird in Kombination mit mindestens einem Nucleosid-Reverse-Transkriptase-Inhibitor.

3. Verwendung nach Anspruch 1 oder 2, wobei die Verbindung 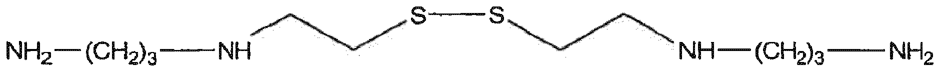 ist.

4. Verwendung nach Anspruch 1 oder 2, wobei die Verbindung 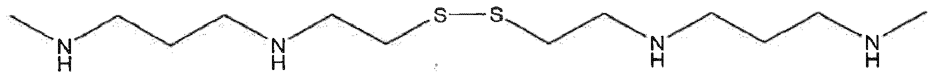 ist.

5. Verwendung nach Anspruch 2, wobei die Nucleosid-Reverse-Transkriptase-Inhibitoren ausgewählt sind aus der Gruppe, bestehend aus Zidovudin, Didanosin, Stavudin, Zalcitabin, Lamivudin, Abacavir und Kombinationen davon.

6. Verwendung nach Anspruch 5, wobei mindestens einer der Nucleosid-Reverse-Transkriptase-Inhibitoren Zidovudin ist.

7. Verwendung nach Anspruch 6, wobei das Zidovudin dem Patienten verabreicht wird in einer täglichen Dosis von 300 mg/Tag bis 400 mg/Tag.

8. Verwendung nach Anspruch 1 oder 2, wobei das Medikament 500 mg bis 700 mg der Verbindung umfasst.

9. Verbindung der Formel (II) oder ein pharmazeutisch annehmbares Salz oder Solvat davon zur Verwendung bei der Behandlung oder Prävention einer HIV-Infektion, wobei die Formel (II) die Struktur 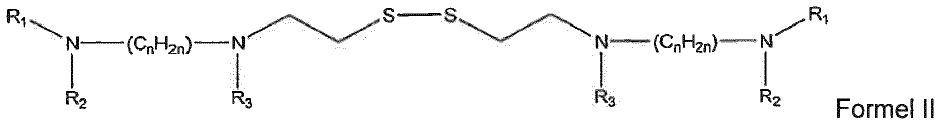 worin jedes von R₁, R₂ und R₃ unabhängig ausgewählt ist aus Wasserstoff und C₁₋₆-Alkyl und worin n eine ganze Zahl von 1 bis 10 ist,
aufweist.

10. Verbindung zur Verwendung nach Anspruch 9, wobei die Verbindung 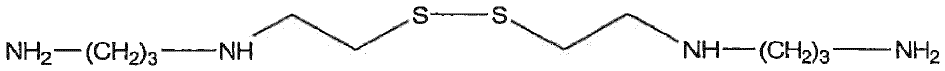 ist.

11. Verbindung zur Verwendung nach Anspruch 9, wobei die Verbindung 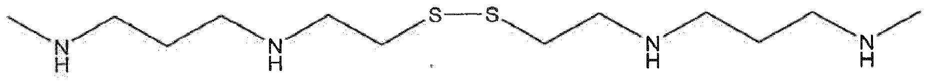 ist.

## Revendications

1. Utilisation d'un composé de formule (II) ou d'un sel ou d'un solvate pharmaceutiquement acceptable de celui-ci dans la préparation d'un médicament destiné au traitement ou à la prévention d'une infection par le virus de l'immunodéficience humaine où la formule (II) a la structure : 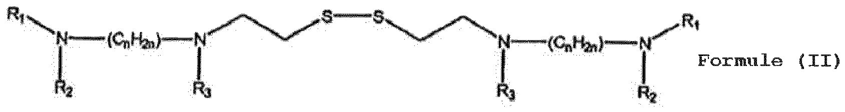 dans laquelle chacun de R₁, R₂ et R₃ est choisi indépendamment parmi un atome d'hydrogène et un groupe alkyle en C₁ à C₆, et dans laquelle n est un nombre entier de 1 à 10.

2. Utilisation selon la revendication 1, dans laquelle ledit médicament est employé en combinaison avec au moins un inhibiteur nucléosidique de la transcriptase inverse.

3. Utilisation selon la revendication 1 ou 2, dans laquelle le composé est

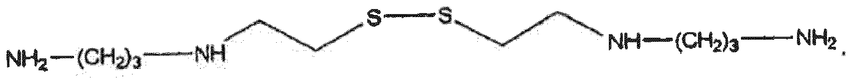

4. Utilisation selon la revendication 1 ou 2, dans laquelle le composé est

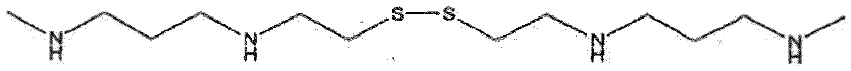

5. Utilisation selon la revendication 2, dans laquelle les inhibiteurs nucléosidiques de la transcriptase inverse sont choisis dans le groupe constitué par la zidovudine, la didanosine, la stavudine, la zalcitabine, la lamivudine, l'abacavir, et des combinaisons de ceux-ci.

6. Utilisation selon la revendication 5, dans laquelle au moins l'un des inhibiteurs nucléosidiques de la transcriptase inverse est la zidovudine.

7. Utilisation selon la revendication 6, dans laquelle la zidovudine est administrée au patient à une posologie quotidienne de 300 mg/jour à 400 mg/jour.

8. Utilisation selon la revendication 1 ou la revendication 2, dans laquelle le médicament comprend de 500 mg à 700 mg du composé.

9. Composé de formule (II) ou un sel ou un solvate pharmaceutiquement acceptable de celui-ci pour une utilisation dans le traitement ou la prévention d'une infection par le virus de l'immunodéficience humaine, où la formule (II) a la structure : 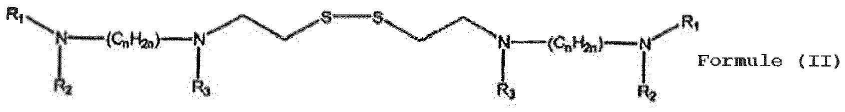 dans laquelle chacun de R₁, R₂ et R₃ est choisi indépendamment parmi un atome d'hydrogène et un groupe alkyle en C₁ à C₆, et dans laquelle n est un nombre entier de 1 à 10.

10. Composé pour une utilisation selon la revendication 9, où le composé est

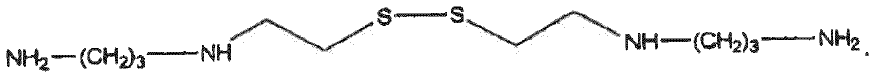

11. Composé pour une utilisation selon la revendication 9, où le composé est
